# EUROPEAN PATENT APPLICATION

(11) **EP 1 480 067 A1**
(43) Date of publication of application: **24.11.2004**
(21) Application number: 04394028.7
(22) Date of filing: 21.05.2004
(51) Int. Cl.: G02B 23/24

(54) **System for indirectly measuring defects**

(30) Priority: 22.05.2003 IL 15607403
(71) Applicant: ISRAEL AIRCRAFT INDUSTRIES, LTD., Lod 70 100 (IL)
(72) Inventor: Bughici, Julian, Hod Hasharon 45272 (IL); Shitzer, Haim, Bnei Brak 51266 (IS)
(74) Representative: Duffy, Assumpta Dympna

(57) **Abstract**

A measurement system for indirectly measuring dimensions of a defect in a target area, within a range of working distances between the measurement system and the target area, the measurement system comprising illuminating optics for illuminating the target area, a laser pattern generator with a laser source and a pattern generating assembly for producing a reference laser pattern having at least one dimension essentially invariant within the range of working distances, the at least one dimension constituting a reference dimension in the target area, and projecting the reference laser pattern, along a laser beam path, onto the target area, and an imaging system with imaging optics for obtaining an image of the target area together with the reference pattern, to determine the linear dimensions of the defect in the image relative to the reference dimension, wherein the laser beam path is spaced apart from the illuminating and imaging optics.

## Description

### FIELD OF THE INVENTION

This invention relates to the measurement of defects in parts of bodies, particularly those parts located in the internal portions of industrial machinery or body organs.

### BACKGROUND OF THE INVENTION

The measurement of defects such as flaws or cracks, etching, corrosion and the like, in the internal parts of machinery (e.g. engines, boilers, turbines, chemical plants, etc.) are commonly performed in order to assess the need for equipment tear down and repair. The visual inspection and measurement of other of inaccessible objects, such as internal body organs is also performed extensively to observe, diagnose and treat internal body organs - determining the size of a tumor or organ wall perforation for example. These visual inspections are performed in order to assess the need for equipment tear down and repair or surgery; thus the results of the inspections are accorded a great deal of importance.

Accordingly, there has been much effort to improve the art in the field of equipment such as borescopes and endoscopes, which are typically used in measurements of this kind.

Endoscopes are optical systems comprising an elongated insertion tube, typically circular in cross-section, which can be inserted through a small opening to give a view of the interior of a body. They almost always include a source of illumination that is conducted along the scope from the outside (proximal) end to the inside (distal) end, so that the interior of the body can be viewed. Endoscopes come in two basic types - flexible endoscopes (fiberscopes and videoscopes) and rigid boroscopes. Flexible scopes are more versatile, but horoscopes can provide higher image quality, are less expensive, easier to manipulate, and are thus generally preferred in those applications for which they are suited.

While endoscopes (both flexible and rigid) can give the user a relatively clear view of an inaccessible region, there is no inherent ability to make a quantitative measurement of the size of the objects being viewed. There are many applications for which the size of an object, such as a tumor in a human body, or a crack in a machine part, is a critically important piece of information.

In U.S. Patent No. 6,476,979 to Schaack, there are disclosed focusing systems for perspective dimensional measurements and optical metrology in which variation of magnification is either inherently compensated, or is calibrated and corrected. The required conditions for inherent compensation are determined and methods for calibrating change in relative magnification and deviation of the optical axis with focal shift are disclosed.

In U.S. Patent No. 6,063,023 to Sakiyama, there is disclosed a measuring endoscope system in which an optical adapter at the distal end of the endoscope has two objective lenses thereby providing two images. These images are formed at different positions on an imaging device of an associated endoscopic unit that calculates three-dimensional coordinates by matching the two images after they have undergone coordinate conversion.

U.S. Patent No. 6,063,023 to Sakiyama discloses an endoscopic apparatus with a guide means that includes a measuring instrument for an endoscope wherein a scale member comprising gradations is attached to the distal end of the apparatus and is caused to abut the part to be measured.

U.S. Patent No. 4,980,763 to Lia discloses, *inter alia,* a device for measuring objects viewed with a boroscope by producing an auxiliary image in conjunction with a pre-calibrated magnification and distance calibration scale overlaid on the object to indicate a modification factor for the measurement. The auxiliary image has an annular form with one known dimension. The boroscope includes a central imaging channel and an illumination source with a plurality of optical fibers surrounding the imaging channel for directing illuminating light onto an object to be measured. The auxiliary image is formed by using a laser light directed onto the object via the same optical fibers as the illuminating light.

GB Patent No. 2,269,453, to Epstein et al, describes an endoscope inspection device having an imaging channel with imaging optics, the laser beam being projected onto the object via the imaging optics to produce a laser pattern having a known dimension. For this purpose, the imaging optics is designed to provide the laser beam with desired characteristics.

### SUMMARY OF THE INVENTION

The present invention relates to an optical apparatus and a method for viewing and measuring defects in internal, essentially inaccessible locations such as within engines or body organs.

In accordance with one aspect of the present invention, there is provided measurement system for indirectly measuring dimensions of a defect in a target area, within a range of working distances between the measurement system and the target area, the measurement system comprising illuminating optics for illuminating the target area, a laser pattern generator with a laser source and a pattern generating assembly for producing a reference laser pattern having at least one dimension essentially invariant within the range of working distances, the at least one dimension constituting a reference dimension in the target area, and projecting said reference laser pattern, along a laser beam path, onto the target area, and an imaging system with imaging optics for obtaining an image of the target area together with the reference pattern, to determine the linear dimensions of the defect in the image relative to the reference dimension, wherein the laser beam path is spaced apart from the illuminating and imaging optics.

The measurement system according to the present invention is particularly advantageous for use with an endoscope, which includes the imaging system. The term *endoscope* will be used hereinafter in the specification and claims to denote all devices for viewing internal chambers including boroscopes, videoscopes, fiberscopes, and the like.

Endoscopes are optical tools that include an elongated insertion tube whose distal end can be inserted into an interior space that is typically inaccessible for direct viewing and measurement. Endoscopes typically further include a source of illumination, and an image acquisition means comprising an imaging system and an image viewing system, whereby the interior space can be viewed directly through an eyepiece and/or displayed on a CRT.

As the endoscope produces an image of the target area that includes the pattern having a known dimension, the dimensions of the defect in the target area can be analyzed and comparatively determined. The comparison can be performed by visual determination, by producing a photograph and measuring, or by image processing means using an appropriate algorithm of a computer software program. In this way, the distance of the distal end of the endoscope from the target area need not be measured, controlled or known in order to achieve accurate and precise measurement of defects.

The reference laser pattern in the system according to the present invention, may be in the form of a circular spot of a known diameter which is essentially invariant within a range of working distances. Such spot may be produced by collimator arrangements known *per se,* e.g. in the field of optical communication.

Alternatively, the reference laser pattern may be in the form of an annular spot or in the form of at least two characters having easily determined centers in which case, the reference dimension will be a distance between these centers. Each such character may be comprised of intersecting of lines, or be a cross and have its center at the intersection points of the lines.

In accordance with another aspect of the present invention, there is provided a measurement apparatus for indirectly measuring a defect within a target area comprising an endoscope having an elongated insertion tube with distal and proximal ends and a laser pattern generator as described above. The laser pattern generator comprises a laser source (adjacent the proximal end of the endoscope), and a pattern generating assembly including a collimator assembly (adjacent the distal end of an endoscope) and a fiber for transmitting the laser energy therebetween. The collimator assembly of the laser pattern generator can be simply attached to the endoscope or, if the endoscope has a so-called *working* *channel,* it can be fixed within the working channel, thus preserving the minimal diameter of the apparatus. Working channels are common to many endoscopes and provide an opportunity for the incorporation of tools, for example a magnet that can be used to retrieve a loose artifact.

In accordance with a still further aspect of the present invention there is provided a method for indirectly measuring a defect within a target area, using a reference laser pattern, as described above, as a basis for comparison with the defect and determination of linear dimensions thereof.

Some advantages of the present invention include:
- The distance of the imaging member from the target need not be known or controlled/adjusted.
- Providing a reference pattern of precise size results in accurate and precise measurements.
- Due to the fact that the laser beam path is spaced apart from the illumination and imaging optics, the laser pattern generator can be easily retrofitted into an existing endoscope. The collimator assembly may be designed to fit within a working channel of the insertion tube of the existing endoscope without the need to modify the endoscope. Alternatively, the collimator assembly may be easily attached to the outside of the insertion tube of the existing endoscope.
- In the case where the collimator assembly of the laser pattern generator is fitted within an existing endoscope, the minimal diameter of the insertion tube is preserved thus preserving the typical easy manipulation and passage of the insertion tube within narrow internal areas.

### BRIEF DESCRIPTION OF THE DRAWINGS

In order to understand the invention and to see how it may be carried out in practice, embodiments will now be described, by way of non-limiting examples only, with reference to the accompanying drawings, in which:
**Fig. 1** is a schematic illustration of a measurement system according to the present invention, in conjunction with an endoscope;
**Fig. 2** is a schematic illustration of the distal end of an alternative design of an endoscope having a working channel, with a laser beam collimator assembly of the laser pattern generator, according to the present invention, installed therein; and
**Figs. 3 - 5** are schematic illustrations of three examples of laser beam collimator assemblies, for producing laser patterns, according to the present invention, wherein:

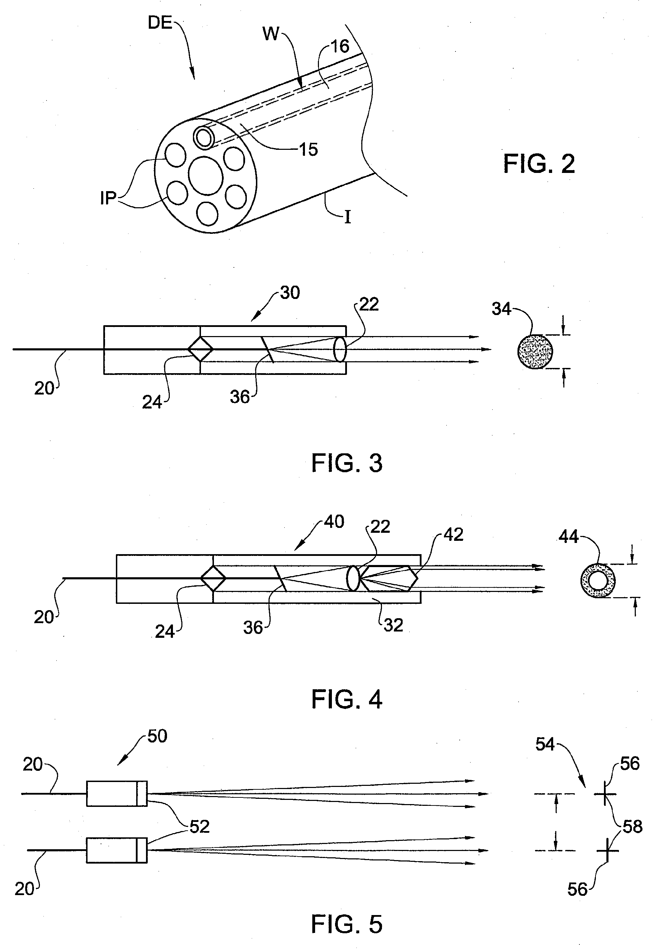

   **Fig. 3** is a laser beam collimator assembly for producing a generally circular laser pattern,
   **Fig. 4** is a laser beam collimator assembly for producing a generally annular laser pattern; and,
   **Fig. 5** is a laser beam collimator assembly for producing a laser pattern comprising two cross-shaped characters.

### DETAILED DESCRIPTION OF THE INVENTION

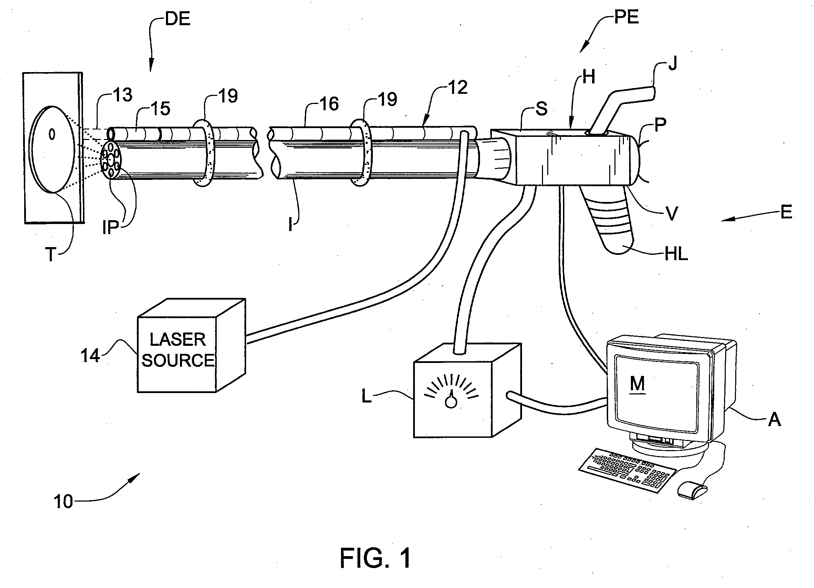

In Fig. **1** is shown a measurement apparatus **10** for indirectly measuring dimensions of a defect in a target area **T** inside a machine to be inspected such as a jet engine, within a range of working distances between the apparatus and the target area.

The apparatus **10** includes an endoscope **E** for imaging the target area **T** and a laser pattern generator **12** for forming a reference laser pattern in the target area. The measurement apparatus **10** may further comprises an image analyzer/processor **A**, which receives signals corresponding to images from the endoscope **E** for automatic determination of the defect measurements based on their comparison with the reference laser pattern.

The endoscope **E** comprises a housing **H** and a flexible insertion tube **I** projecting therefrom for insertion into the machine to be inspected via an inspection port or opening therein, the tube having a proximal end **PE** associated with the housing **H**, and a distal end **DE**.

The endoscope comprises an optical scope **S** with optics located inside the insertion tube **I** and the housing **H** that forms an image of the target area **T** via the distal end **DE** of the insertion tube **I**, and enables viewing thereof by a user's eye or by a video camera. For this purpose, the endoscope **E** may respectively incorporate an eyepiece **P** and/or an image-to-video converter (not seen) located within the housing **H**. The endoscope may include components such as a CCD camera, enabling the display of images obtained from the scope **S**, on a display monitor **M** or processing the image by the image analyzer/processor **A**.

The endoscope **E** further comprises a light source **L** for the illumination of the target area **T** via optical fibers (not seen) extending along the insertion tube **I** and terminating at illumination ports **IP**.

The endoscope **E** may further comprise a joystick **J** for manipulating the insertion tube **I** to bring its distal end into an appropriate orientation with respect to the target area **T.** The endoscope **E** may further comprise a handle **HL** for the convenience of its operation.

The laser pattern generator **12** comprises a laser source **14** and a laser beam-shaping device **16** designed to form a desired reference laser pattern from a beam of radiation emitted by the laser source **14**. The reference laser pattern is projected along a laser beam path **13** that is spaced apart from the optics of the scope **S** and the illumination ports **IP**. The laser source **14** can be a diode laser (such as a single mode diode laser) or any other type that is capable of providing energy of the desired power, i.e. that produces a laser pattern that is visible and such that it does not damage the part to be measured.

The laser beam-shaping device **16** is in the form of a conduit with suitable optical components housed at its head portion **15** and a laser radiation guide, such as an optic fiber, via which a beam of laser radiation is guided to the head portion to be shaped therein by the optical components into a desired reference laser pattern, which will be described in more detail with reference to Figs. **3** - **5.**

In Fig. **1**, the conduit of the laser beam-shaping device **16** of the laser pattern generator **12** is strapped to the insertion tube **I** of the endoscope **E** by straps **19.**

In Fig. **2,** an alternative arrangement is illustrated, where the endoscope **E** comprises within its insertion tube **I** a working channel **W** with the laser beam-shaping device **16** incorporated therein.

Referring now to Figs. **3 - 5,** three examples of the laser beam-shaping device **16** will be described, each comprising a collimator assembly **30, 40** or **50,** and an optical fiber **20**, for example a single mode patch fiber, held in place by a holding member **24**, for directing into the assembly a laser beam. In all three examples, a laser reference pattern is formed by the laser pattern generator **12** in which at least one dimension (reference dimension) is invariant within the range of working distances of the apparatus **10**.

Fig. **3** shows a collimator assembly **30** comprises a collimator lens **22,** preferably in the form of an advanced technology lens such as a Gradium™ (axial gradient index glass) lens (Lightpath Technologies, Albuquerque, N.M., USA), which shapes the laser beam into a circular spot pattern **34** having an invariant diameter within the range of working distances. For clarification, the laser will keep its generally circular shape at the target area **T** when the pattern **34** laser beam is perpendicular to the target area **T**. However, if the laser beam were projected onto the target area **T** at an angle, the pattern **34** would tend to be somewhat elliptical and would have at least one dimension invariant - the minor axis of the elliptical pattern.

To preclude the reflection of laser energy back into the fiber **20**, which can harm the system, the end of the fiber may be cut at an angle as shown at **36**.

Fig. **4**, shows a collimator assembly **40** that is generally similar to that of the assembly described in reference to Fig. **3**, however the assembly further comprises a double-coned lens **42,** thereby producing a pattern **44** in the form of an annular spot. An advantage to such a pattern **44** is that its energy density is more uniformly distributed. The most convenient invariant dimension in this case is deemed to be the outer diameter of the pattern **44**, however the inner diameter and the annular width is also invariant.

Fig. **5** shows a collimator assembly **50** illustrating another means of producing a pattern that can provide an invariant dimension. Here, the assembly comprises two optical lines each associated with a laser source, such as laser source **14**, and each having a collimator lens **52** to which a laser radiation beam is directed via an optical fiber such as in the assemblies **30** and **40** described above.

The collimator lenses **52** are preferably small lenses, such as gradient index (GRIN) lenses, known *per se,* in order to preserve space such that the two collimator lenses fit in the working channel **W.** The two lenses **52** are precisely and parallely disposed to produce a pattern **54** comprised of, for example, two cross-shaped characters **56** whose centers **58** are spaced apart to a predetermined distance. The distance between the centers **58** is thus invariant within the range of working distances and can therefore be used as the reference dimension. The laser beam characters **56** shown in Fig. **5** may be produced by means of a masking arrangement (i.e. whereby a mask or covering is used having a cutout in the form of a cross).

The term "invariant" in the above examples means that the divergence of the laser beam, or the distance between the centers **58** of characters such as characters **56**, within the range of working distances, does not exceed the desired accuracy of measurements of the defect.

A divergence of at least as small as about 0.35 mRad can be achieved in a laser produced by a laser device of the kind described above in conjunction with Figs. 3 and 4 with the above-referred to equipment, thereby maintaining a maximum pattern deviation of +/- 0.01 mm in a working range of 3 cm, with a device having, for example, the following specifications:
- Single mode diode laser: Wavelength = 660 nm, Power range: 6.0 - 9.0 mW, Laser driver current = 250 mA;
- Single mode fiber: Wavelength = 660 nm, Mode field diameter = 4.0 µm (0.004 mm), Attenuation (typical) = 10 db/km, Natural attenuation = 0.12 db/km;
- Collimating Gradium™ lens: Gradium™ material: glass (Hoya company, Japan) TAC4, Effective Focal Length (EFL) = 5.0 mm, Back Focal Length (BFL) = 4.36 mm, Natural attenuation = 0.30 db/km, Design wavelength = 780 nm.

In operation of the measuring apparatus **10**, with any of the collimator assemblies **30, 40,** or **50** described above, the invariant dimension of the laser reference pattern **34, 44,** or **54** is first measured outside the machine to be inspected (this measurement only required once), the endoscope's insertion tube **I** with the laser beam-shaping device **16** is inserted into the machine to be inspected via an inspection port or opening therein, the target area **T** with the reference pattern is imaged by the endoscope **E** and linear dimensions of defects are determined based on the comparison thereof in the obtained image with the reference dimension of the reference pattern.

Thus, the above described measuring system and apparatus is one that provides an essentially unobtrusive, convenient, accurate and precise arrangement for measuring defects in generally inaccessible locations.

It should be understood that there are various measuring systems/apparatus for indirectly measuring defects that can be devised according to the present invention and that the above descriptions are merely explanatory. Thus, the measuring systems/apparatus can be embodied in a variety of aspects falling within the scope of the present invention, *mutatis mutandis.*

## Claims

1. A measurement system for indirectly measuring dimensions of a defect in a target area, within a range of working distances between the measurement system and the target area, the measurement system comprising illuminating optics for illuminating said target area, a laser pattern generator with a laser source and a pattern generating assembly for producing a reference laser pattern having at least one dimension essentially invariant within the range of working distances, the at least one dimension constituting a reference dimension in the target area, and projecting said reference laser pattern, along a laser beam path, onto the target area, and an imaging system with imaging optics for obtaining an image of the target area together with the reference pattern, to determine the linear dimensions of the defect in the image relative to the reference dimension, wherein said laser beam path is spaced apart from said illuminating and imaging optics.

2. The measurement system according to claim 1, wherein the imaging system further comprises a camera for displaying and/or recording the image of the target area.

3. The measurement system according to Claim **1**, wherein the imaging system comprises an eyepiece for viewing the image.

4. The measurement system according to claim **1**, wherein the laser pattern generator comprises collimator optics.

5. The measurement system according to claim **4**, wherein the collimator optics are at the distal end of the laser pattern generator.

6. The measurement system according to claim **4**, wherein the distal end of the laser pattern generator is disposed at the distal end of the measurement system.

7. The measurement system according to claim **4**, wherein the reference pattern is circular.

8. The measurement system according to claim **4**, wherein the reference pattern is annular.

9. The measurement system according to claim **8**, wherein the laser pattern generator comprises a double-coned lens.

10. The measurement system according to claim **4**, wherein the reference pattern comprises two characters whose centers are of a predetermined distance from each other.

11. The measurement system according to claim **10**, wherein the laser pattern generator comprises two optical lines each generating one of the characters.

12. The measurement system according to claim **11**, wherein the characters are cross-shaped and each of the optical lines comprises a gradient index (GRIN) lens.

13. The measurement system according to claim **1**, wherein the imaging system is insertable in an endoscope having a working channel spaced from its optical axis, and the pattern generating assembly is housed in a conduit which is adapted to be integratable within said working channel.

14. The measurement system according to claim **1**, wherein said measurement system further comprises an image analyzer/processor for automatically determining the size of the defect.

15. A method for indirectly measuring dimensions of a defect in a target area, including:
- illuminating said target area;
- producing a reference laser pattern having at least one dimension essentially invariant within the range of working distances, the at least one dimension constituting a reference dimension in the target area;
- projecting said laser beam pattern, along a laser beam path, into the target area;
- imaging the target area together with the reference pattern;
- determining linear dimensions of the defect in the image of the target area by comparing them with the reference dimension.

16. The method according to claim **15**, wherein the linear dimensions of the defect are determined by a visual comparison of the laser pattern and the defect in the image.

17. The method according to claim **15**, wherein the linear dimensions of the defect are determined by measurement of the image of the target area that includes the laser pattern and the defect.

18. The method according to claim **17**, wherein the image is depicted in a photograph.

19. The method according to claim **17**, wherein the image is depicted on a display monitor.

20. The method according to claim **15**, wherein the linear dimensions of the defect are determined by analysis using a processor comprising an algorithm for same.

21. The method according to claim **15**, wherein the range of working distances is at least 3 cm.

22. The method according to claim **15**, wherein the reference dimension is considered to be invariant if its change within the range of working distances does not exceed 0.01 mm.

23. The method according to claim **15**, wherein the reference pattern is in the form of either a circular or annular spot.

24. The method according to claim **15**, wherein the reference pattern is in the form of two characters whose centers are of a predetermined distance from each other.

25. A measurement apparatus for indirectly measuring a defect within a target area, comprising an endoscope and a measurement system according to any of claims **1-14.**
